(19) Europäisches Patentamt
European Patent Office
Office européen des brevets

(11) Publication number: **0 170 350**
**B1**

(12) **EUROPEAN PATENT SPECIFICATION**

(45) Date of publication of patent specification: **19.07.89**

(51) Int. Cl.[4]: **C 07 F 5/02,** C 07 D 249/08

(21) Application number: **85303017.9**

(22) Date of filing: **29.04.85**

(54) **An asymmetrically modified boron hydride type compound, a production method thereof, and a method for producing an optically active alcohol derivative by the use thereof.**

(30) Priority: **06.07.84 JP 140963/84**
**12.09.84 JP 191170/84**

(43) Date of publication of application:
**05.02.86 Bulletin 86/06**

(45) Publication of the grant of the patent:
**19.07.89 Bulletin 89/29**

(84) Designated Contracting States:
**BE CH DE FR GB IT LI NL**

(56) References cited:
**EP-A-0 054 431**
**WO-A-84/03885**

**JOURNAL OF THE CHEMICAL SOCIETY, Perkin I, 1981, pages 231-235, London, GB; M.F. GRUNDON et al.: "Asymmetric induction. Part 3.1,2. Assymmetric reduction of ketones with amine-boranes in the presence of acids"**

(73) Proprietor: **SUMITOMO CHEMICAL COMPANY, LIMITED**
**Kitahama 4-chome 5-33**
**Chuo-ku Osaka 541 (JP)**

(72) Inventor: **Yoneyoshi, Yukio**
**30-16, Hiyoshidai-3-chome**
**Otsu-shi (JP)**
Inventor: **Suzukamo, Gohfu**
**1-216, Kuwatacho-2-chome**
**Ibaraki-shi (JP)**
Inventor: **Hamada, Kazuhiko**
**15-23, Oeminamifukunishicho-2-chome**
**Nishigyo-ku Kyoto (JP)**
Inventor: **Nishioka, Toshio**
**15-10-405, Kusunokicho**
**Ashiya-shi (JP)**

(74) Representative: **Harrison, David Christopher et al**
**MEWBURN ELLIS & CO 2/3 Cursitor Street**
**London EC4A 1BQ (GB)**

**EP 0 170 350 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an asymmetrically modified boron hydride type compound, its production method and a method for producing an optically active alcohol derivative using the compound. More particularly, it relates to an asymmetrically modified boron hydride type compound obtained by reacting an optically active amino alcohol of the formula:

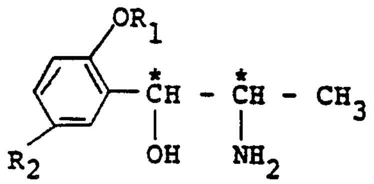

(I)

wherein $R_1$ is a $C_{1-3}$ alkyl group, $R_2$ is a hydrogen atom, a $C_{1-3}$ alkoxyl group or a $C_{1-4}$ alkyl group and a mark * means an asymmetric carbon with the proviso that $R_2$ is not an ethoxy group when $R_1$ is an ethyl group, or its salt with an acid, with a boron hydride compound; its production method; and a method for producing an optically active alcohol derivative of the formula:

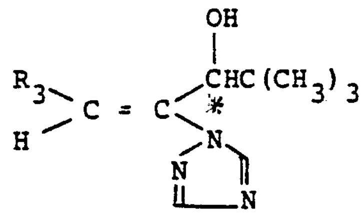

(II)

wherein $R_3$ is a cycloalkyl group or a phenyl group which may be substituted with at least one of halogen and a mark * has the same meaning as above, by carrying out the asymmetric reduction of a ketone compound of the formula:

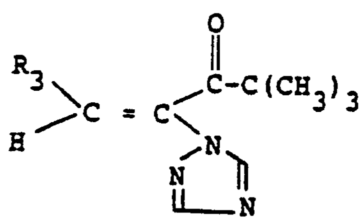

(III)

wherein $R_3$ has the same meaning as above, with said asymmetrically modified boron hydride type compound.

The alcohol derivative of the above formula (II), i.e. an azole type α,β-unsaturated alcohol derivative, is known to be useful as an active ingredient for fungicides, plant growth regulators or herbicides, as represented for example by 1 - (2,4 - dichlorophenyl) - 2 - (1,2,4 - triazol - 1 - yl) - 4,4 - dimethyl - 1 - penten - 3 - ol, 1 - (4 - chlorophenyl) - 2 - (1,2,4 - triazol - 1 - yl) - 4,4 - dimethyl - 1 - penten - 3 - ol and 1 - cyclohexyl - 2 - (1,2,4 - triazol - 1 - yl) - 4,4 - dimethyl - 1 - penten - 3 - ol [JP—A—124771/1980, 25105/1981 and 111477/1980].

Furthermore, it is also well known that there is a remarkable difference in activity between the optical isomers, and that, for example, with reference to the foregoing alcohol derivatives the (−)-isomers have a strong activity as fungicides, while the (+)-isomers have a strong activity as plant growth regulators and herbicides [JP—A—99575/1982, 106669/1982, 139370/1983 and 139371/1982].

The biological activities of the above-described optical isomers can be enhanced by raising their optical purities, whereby the amounts of chemicals to be applied can be decreased to avoid harmful effects upon the environment. For example, when the above-described alcohol derivatives are used as fungicides, a high optical purity of (−)-isomer becomes necessary because of phytotoxicity caused by the plant growth regulating activity of (+)-isomer.

For this reason, there is a great demand for the development of a method which allows production of either one of the (−)- and (+)-optical isomers having an extremely high optical purity required for the intended uses and yet which can be carried out efficiently on an industrial scale.

A method for reducing the carbonyl group of ketone compounds of the above formula (III) to obtain alcohol compounds of the above formula (II) using asymmetrically modified borohydride compounds is described in WO 84/03885, published after both of the priority dates of this application. This document is acknowledged under Art 54(3) EPC.

The applicants' own EP—A—0171175 claims priority from Japanese Patent Application No. 140157/84, which application contains disclosure present in EP—A—0171175, and which disclosure in the Japanese

application is acknowledged under Art 54(3) EPC. A method for the production of an optically active borane complex and its use in the reduction of prochiral ketone compounds are described. The method consists of reacting the acid salt of norephedrine with a metal borohydride, followed by hydrolysis of the resultant product to obtain the borane complex, which has the formula

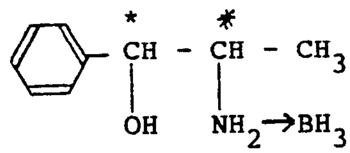

We have conducted extensive investigations into methods for obtaining an alcohol derivative of the formula (II) of greater optical purity by the asymmetric reduction of the ketone compound of the above formula (III), and as a result, found that, by using an asymmetrically modified boron hydride compound (hereinafter referred to as present compound) obtained by reacting the optically active amino alcohol of the above formula (I) having a 2-alkoxyl group in the phenyl group thereof or its salt with an acid with a boron hydride compound, only the carbonyl group is selectively reduced into the desired optically active alcohol derivative in a high optical yield.

Next, the present invention will be illustrated.

In the optically active amino alcohol of the above formula (I), a starting material for production of a borane complex in accordance with the invention (hereinafter "the present compound"), specific examples of a substituent $R_1$ include a methyl group, an ethyl group or a propyl group. Specific examples of $R_2$ include a hydrogen atom, a methoxy group, an ethoxy group, a propoxy group, a methyl group, an ethyl group or a tert-butyl group. More specifically, as the optically active amino alcohol of the formula (I), there may be given 1-(2,5 dimethoxyphenyl)-2-amino-1-propanol, 1-(2,5 dipropoxyphenyl)-2-amino-1-propanol, 1-(2-methoxyphenyl)-2-amino-1-propanol, 1-(2-ethoxyphenyl)-2-amino-1-propanol, 1-(2-propoxyphenyl)-2-amino-1-propanol, 1-(2-methoxy-5-methylphenyl)-2-amino-1-propanol, 1-(2-methoxy-5-ethylphenyl)-2-amino-1-propanol and 1-(2-ethoxy-5-methylphenyl)-2-amino-1-propanol. These optically active amino alcohols are produced, for example, by the methods described in W. H. Hartung, et al., J. Am. Chem. Soc., *52*, 3317—22 (1930); W. H. Hartung, et al., J. Am. Chem. Soc., *53*, 4149—60 (1931); R. Baltzly, et al., J. Med., *11* (4), 833—44 (1968); US—A—2,359,707, and JP—A—39648/1983.

In the present invention, the halogen atom represents fluorine atom, chlorine atom or bromine atom.

Next, reference will be made to a method for producing the present compound.

The present compound, when the boron hydride compound is a metal borohydride, may be obtained by reacting a salt, as obtained from the optically active amino alcohol of the formula (I) and an acid, with the metal borohydride in a solvent, or when the boron hydride compound is a borane, it is obtained by directly reacting the optically active amino alcohol of the formula (I) with the borane in a solvent. As the foregoing acid which is a starting material for producing the salt of the optically active amino alcohol, there are given mineral acids (e.g. hydrochloric acid, sulfuric acid, nitric acid, phosphoric acid), carboxylic acids (e.g. acetic acid) and organic sulfonic acids (e.g. p-toluenesulfonic acid). The salt may be used as such or may be produced, in situ, from the optically active amino alcohol and the acid in the reaction system for producing the reducing agent to be used.

As the metal borohydride described above, there are given for example sodium borohydride, potassium borohydride, lithium borohydride and zinc borohydride. Generally, however, the object of the present invention can be achieved sufficiently well by using easily available sodium borohydride. Examples of the borane are diborane, borane-tetrahydrofuran complex and, borane-dimethyl sulfide complex.

In production of the present compound, the molar ratio of the boron hydride compound to the optically active amino alcohol may be, when the boron hydride compound is a metal borohydride, 0.7:1 to 2:1, preferably 0.7:1 to 1.3:1, more preferably 1 to 1, as converted to boron basis, and when said compound is a borane, the molar ratio may be 0.7:1 to 1.3:1, preferably 1 to 1.

The solvent used in producing the present compound is not particularly limited, so long as it does not take part in the reaction. For example, however, there are given aromatic hydrocarbons (e.g. benzene, toluene, xylene, chlorobenzene), halogenated hydrocarbons (e.g. methylene chloride, 1,2-dichloroethane, chloroform, carbon tetrachloride), and mixtures thereof. When the metal borohydride is used, in order to dissolve it, for example, dimethyl sulfoxide diglyme, dimethylformamide or 1,3-dimethyl-2-imidazolidinone may be used in combination with the abovementioned solvent. The reaction temperature is generally within a range of −78° to 100°, preferably −40° to 50°C. The reaction is generally carried out in an inert gas atmosphere such as nitrogen or argon.

The present compound thus obtained may be used for the subsequent reduction after its separation from the reaction solution, but generally, it is used as a solution without prior separation from the reaction solution.

Next, reference will be made to a method for producing the optically active alcohol derivative of the above formula (II) by reduction of the ketone compound of the above formula (III) using the present compound thus obtained.

The amount of the present compound used in the reduction is not less than 0.5 mole, generally within a range of 1 to 5 moles, as converted to boron basis, based on 1 mole of the ketone compound of the formula (III), and even the range of 1 to 2 moles can sufficiently achieve the object.

Also, the solvent used in the foregoing reduction is not particularly limited, so long as it is an inactive solvent. Preferably, however, organic solvents such as aromatic hydrocarbons (e.g. benzene, toluene, xylene, chlorobenzene), halogenated hydrocarbons (e.g. methylene chloride, 1,2-dichloroethane, chloroform, carbon tetrachloride), ethers (e.g. diethyl ether, tetrahydrofuran, dioxane, diglyme) and mixtures thereof are used. Also, the solvent used in producing the present compound may be used as it is or in mixture with the solvents described above. The reduction is generally carried out in an inert gas atmosphere as described above. The temperature of the reduction is generally within a range of −30° to 100°C, and industrially within a range of −10° to 50°C.

The foregoing reduction may be carried out in the presence of an acid, and particularly when sodium borohydride is used as a material for producing the present compound, isomerization between the E-form and Z-form of the ketone compound of the above formula (I) is inhibited, whereby the yield of the desired optically active alcohol derivative can be increased. As the acid, there are given for example Lewis acids (e.g. titanium tetrachloride, boron trifluoride etherate, aluminum chloride), carboxylic acids (e.g. acetic acid, chloroacetic acid, propionic acid) and mineral acids (e.g. hydrochloric acid, sulfuric acid, phosphoric acid). The molar ratio of these acids to the ketone compound is generally within a range of 0.01:1 to 1:1, preferably 0.01:1 to 0.5:1.

After the reduction is carried out in this way, the aqueous solution of a mineral acid (e.g. hydrochloric acid, sulfuric acid) is generally added to the reaction solution, the organic layer is separated from the aqueous layer, washed with water and dried, and then the organic solvent is removed by evaporation. By this procedure, the desired aforementioned optically active alcohol derivative of the formula (II) is obtained in a high yield.

The optical purity is determined by measuring the optical rotation of the product obtained, or directly measuring the enantiomer ratio by high-performance liquid chromatography with optically active packing materials.

Hereupon, the optically active amino alcohol used can easily be recovered, with its steric configuration maintained, by adding an aqueous alkali solution to the aqueous layer after the reaction and extracting with an organic solvent. The recovered optically active amino alcohol can be re-used.

Example 1

In a nitrogen atmosphere, 0.223 g (0.9 mmole) of (+)-1-(2,5-dimethoxyphenyl)-2-amino-1-propanol hydrochloride was suspended in 2.5 ml of deutero chloroform, and after cooling to −30°C, a solution of 0.034 g (0.9 mmole) of sodium borohydride in 1 ml of dimethylformamide was added. The temperature of the resulting mixture was raised from −30°C to room temperature over 2.5 hours, to obtain a solution of the present compound. $^{11}B$ nuclear magnetic resonance spectrum (Varian Company-made, Type XL-200, standard, $BF_3 \cdot OEt_2$) of this solution was as follows: −21.1 ppm +6.0 ppm.

Examples 2 and 3

Reaction was carried out in the same manner as in Example 1 except that (+)-1-(2,5-dimethoxyphenyl)-2-amino-1-propanol hydrochloride was replaced by (+)-1-(2-methoxyphenyl)-2-amino-1-propanol hydrochloride or 1-(2-ethoxyphenyl)-2-amino-1-propanol hydrochloride, to obtain a solution of the present compound having the physical property shown in Table 1.

Table 1

| Example No. | $^{11}B$ NMR spectrum [δ (ppm)] |
|---|---|
| 2 | -20.8 +6.2 |
| 3 | -21.1 +6.5 |

Example 4

In a nitrogen atmosphere, 0.392 g (1.8 mmoles) of (+)-1-(2-methoxyphenyl)-2-amino-1-propanol hydrochloride was suspended in 5 ml of 1,2-dichloroethane, and after cooling to −25°C, a solution of 0.068 g (1.8 mmoles) of sodium borohydride in 1 ml of dimethylformamide was added. The temperature of the resulting suspension was raised from −25°C to room temperature over 3 hours. Thereafter, a solution of

0.39 g (1.2 mmoles) of (E)-1-(2,4-dichlorophenyl)-2-(1,2,4-triazol-1-yl)-4,4-dimethyl-1-penten-3-one (E/Z=97.6/2.4) in 4 ml of 1,2-dichloroethane was added to this suspension at 20°C, and then stirring was carried out for 69 hours. Thereafter, 8 ml of 2N hydrochloric acid was added, followed by stirring. The organic layer was washed with water, dried and concentrated under reduced pressure. The residue was purified on a column packed with 2 g of silica gel with a chloroform solvent and then concentrated under reduced pressure to obtain 0.39 g of (−)-(E)-1-(2,4-dichlorophenyl)-2-(1,2,4-triazol-1-yl)-4,4-dimethyl-1-penten-3-ol as a crude crystal. By gas-chromatographic analysis, it was found that the conversion was 100%, and the composition of the reaction product was: E-form alcohol, 96.2%, Z-form alcohol, 3.7% (Z-form alcohol was produced through isomerization of the ketone compound to the Z-form, followed by reduction of the carbonyl group) and a saturated alcohol, less than 0.1% (the saturated alcohol of the reaction products means a product obtained by hydrogenation of both the carbonyl group and the carbon/carbon double bond contained in the ketone compound which is a starting material).

By high-performance liquid-chromatographic analysis using an optically active column, it was found that the enantiomer ratio of the E-form alcohol was: (−)-isomer, 96.6% and (+)-isomer, 3.4%. The optical yield was 93.2%.

### Examples 5 to 10

Reaction was carried out according to Example 4 using the amino alcohol hydrochloride and ketone compounds described below. The results are shown in Table 2.

### Example 11

In a nitrogen atmosphere, a solution of 0.380 g (1.8 mmoles) of (+)-1-(2,5-dimethoxyphenyl)-2-amino-1-propanol in 4 ml of 1,2-dichloroethane was added dropwise at −78°C to a mixture comprising 1.8 ml of a 0.500M diborane-tetrahydrofuran solution and 2 ml of 1,2-dichloroethane, and the temperature of the resulting mixture was raised from −78°C to room temperature over about 2 hours. Thereafter, to this solution was added dropwise at room temperature a solution of 0.39 g (1.2 mmoles) of (E)-1-(2,4-dichlorophenyl)-2-(1,2,4-triazol-1-yl)-4,4-dimethyl-1-penten-3-one (E/Z=97.6/2.4) in 4 ml of 1,2-dichloroethane, and stirring was carried out for 24 hours. Thereafter, 8 ml of 2N hydrochloric acid was added to the reaction solution, followed by stirring for about 2 hours. The organic layer was washed with water and concentrated under reduced pressure. The residue was purified on a column packed with 2 g of silica gel using a chloroform solvent and then concentrated under reduced pressure to obtain 0.39 g of (−)-(E)-1-(2,4-dichlorophenyl)-2-(1,2,4-triazol-1-yl)-4,4-dimethyl-1-penten-3-ol as a crude crystal. The conversion was 98.4%, and the composition of the product obtained was: E-form alcohol, 97.1% and Z-form alcohol, 2.9%. The enantiomer ratio of the E-form alcohol was: (−)-isomer, 95.1% and (+)-isomer, 4.9%. The optical yield was 90.2%.

### Example 12

Reaction was carried out in the same manner as in Example 11 except that (E)-1-cyclohexyl-4,4-dimethyl-2-(1,2,4-triazol-1-yl)-1-penten-3-one (E/Z=99.9/0.1) was used in place of (E)-1-(2,4-dichlorophenyl)-2-(1,2,4-triazol-1-yl)-4,4-dimethyl-1-penten-3-one. The conversion was 100%, and the composition of the product obtained was: E-form alcohol, 99.7% and Z-form alcohol, 0.3%. The anantiomer ratio of the E-form alcohol was: (+)-isomer, 87.8% and (−)-isomer, 13.2%. The optical yield was 74.6%.

### Comparative Examples 1 to 7

Reaction was carried out according to Example 4 using the amino alcohol hydrochloride and ketone compounds described below. The results are shown in Table 3.

Table 2

| Example No. | Ketone compound | Amino alcohol |
|---|---|---|
| 5 | 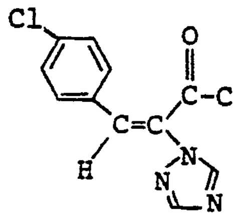 (E/Z=98.9/1.1) | (+) 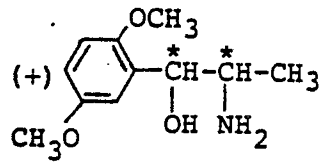 |
| 6 | " ( " ) | (+) 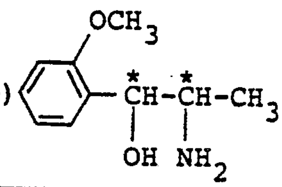 |
| 7 | 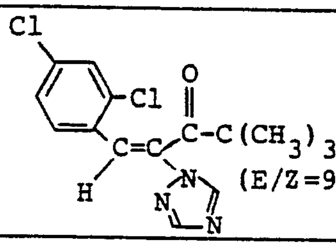 (E/Z=97.6/2.4) | (+) 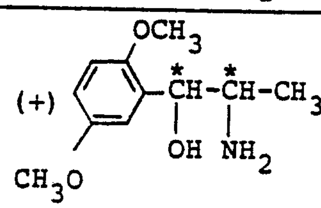 |
| 8 | " ( " ) | (-) 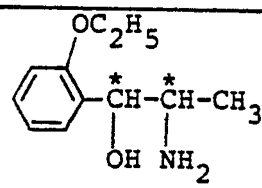 |
| 9 | 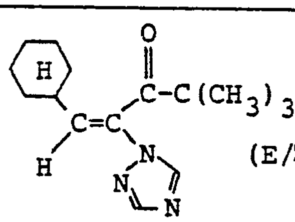 (E/Z=99.9/0.1) | (+) 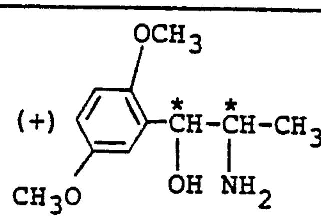 |
| 10 | 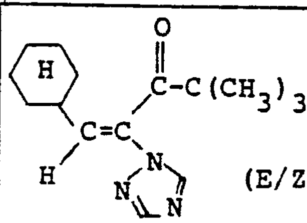 (E/Z=99.9/0.1) | (-) 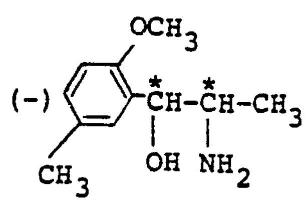 |

Table 2 (Cont'd)

| Reaction temperature (°C) | Reaction time (hr) | Conversion (%) | Reaction product | | | Enantiomer ratio (-/+) of E-form alcohol | Optical yield of E-form alcohol (%) |
|---|---|---|---|---|---|---|---|
| | | | E-form alcohol (%) | Z-form alcohol (%) | Saturated alcohol (%) | | |
| 10-12 | 98 | 94.2 | 96.2 | 3.7 | <0.1 | 98.1/1.9 | 96.2 |
| 20 | 69 | 98.2 | 90.5 | 9.4 | <0.1 | 97.3/2.7 | 94.6 |
| 20 | 24 | 100 | 97.0 | 3.0 | <0.1 | 94.7/5.3 | 89.4 |
| 20 | 21 | 99.0 | 96.4 | 3.5 | <0.1 | 6.6/93.4 | 86.8 |
| 20 | 20 | 100 | 97.2 | 2.8 | - | 13.0/87.0 | 74.0 |
| 20 | 43 | 100 | 97.0 | 3.0 | - | 86.7/13.3 | 73.4 |

Table 3

| Comparative Example No. | Ketone compound | Amino alcohol |
|---|---|---|
| 1 | 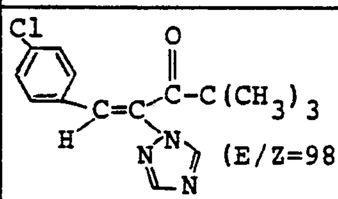 (E/Z=98.9/1.1) | (+) 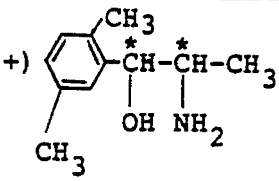 |
| 2 | " ( " ) | " |
| 3 | " (E/Z=99.8/0.2) | (+) 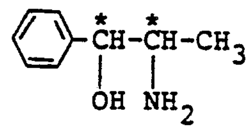 |
| 4 | 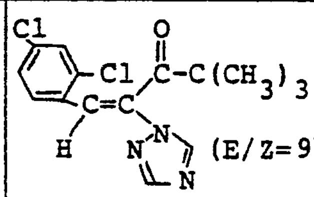 (E/Z=97.6/2.4) | (+) 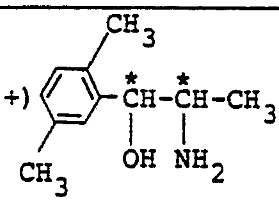 |
| 5 | " (E/Z=99.9/0.1) | (+) 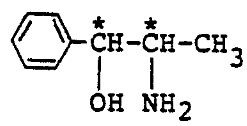 |
| 6 | 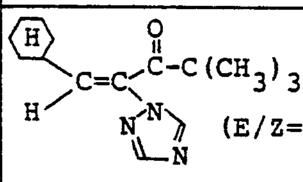 (E/Z=99.9/0.1) | (+) 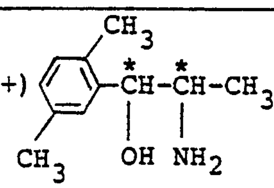 |
| 7 | " | (-) 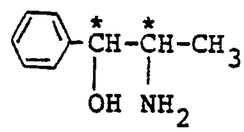 |

- Cont'd -

Table 3 (Cont'd)

| Reaction temperature (°C) | Reaction time (hr) | Conversion (%) | Reaction product: E-Form alcohol (%) | Z-form alcohol (%) | Satu- alcohol (%) | Enantiomer ratio (-/+) of E-form alcohol | Optical yield of E-form alcohol (%) |
|---|---|---|---|---|---|---|---|
| 10-12 | 98 | 92.6 | 95.0 | 4.9 | <0.1 | 96.1/3.9 | 92.2 |
| 20 | 69 | 98.7 | 93.6 | 6.3 | <0.1 | 95.9/4.1 | 91.8 |
| 20 | 24 | 97.5 | 83.7 | 15.9 | 0.4 | 81.1/18.9 | 62.2 |
| 20 | 69 | 99.9 | 96.6 | 3.3 | <0.1 | 90.2/9.8 | 80.4 |
| 20 | 23 | 96.4 | 98.3 | 1.7 | - | 85.1/14.9 | 70.2 |
| 20 | 24 | 100 | 97.1 | 2.9 | - | 19.1/80.9 | 61.8 |
| 20 | 20 | 99.5 | 94.9 | 5.1 | - | 79.7/20.3 | 59.4 |

Reference Example 1

A mixture of 14.05 g of (±)-erythro-2-amino-1-(2-methoxyphenyl)-1-propanol (erythro/threo=98.0/2.0), 10.09 g (of D-(−)-pantolactone and 100 ml of water was heated for 1 hour with stirring and concentrated under reduced pressure. The residue obtained was recrystallized from 110 ml of isopropanol to obtain 5.09 g of the D-(−)-pantoic acid salt of (+)-erythro-2-amino-1-(2-methoxyphenyl)-1-propanol $[\alpha]_D$: +22.9° (c=0.9, water). This diastereomer salt was decomposed with a solution of 1.61 g of potassium hydroxide in 20 ml of water and extracted with chloroform to obtain 2.58 g of (+)-erythro-2-amino-1-(2-methoxyphenyl)-1-propanol. By dissolving this aminoalcohol in diethyl ether-chloroform mixtures and passing hydrogen chloride gas therethrough, the hydrochloric acid salt of the aminoalcohol was formed. This salt was collected by filtration and dried to obtain 2.82 g of (+)-erythro-2-amino-1-(2-methoxyphenyl)-1-propanol hydrochloride. $[\alpha]_D$: +26.5° (c=1.0, water). This product was converted to its sugar derivative (diasteromer) and analyzed by high-performance liquid chromatography to find that its optical purity was 62.6%. The filtrate after recrystallization was concentrated under reduced pressure to obtain 20.28 g of the D-(−)-pantoic acid salt of (−)-erythro-2-amino-1-(2-methoxyphenyl)-1-propanol. $[\alpha]_D$: +1.29° (c=1.0, water). The product was recrystallized from isopropanol, and after filtraton, the filtrate obtained was concentrated under reduced pressure, decomposed with a solution of 3.76 g of potassium hydroxide in 47 ml of water and extracted with chloroform to obtain 6.79 g of (−)-erythro-2-amino-1-(2-methoxyphenyl)-1-propanol. This aminoalcohol was converted to its sugar derivative (diastereomer) and analyzed by high-performance liquid chromatography to find that its optical purity was 44.6%. By dissolving this aminoalcohol in diethyl ether-chloroform mixture and passing hydrogen chloride gas therethrough, (−)-erythro-2-amino-1-(2-methoxyphenyl)-1-propanol hydrochloride was obtained. After recrystallizing this hydrochloride three times from ethanol, 1.75 g of (−)-erythro-2-amino-1-(2-methoxyphenyl)-1-propanol hydrocchloride was obtained from the filtrate. $[\alpha]_D$: −37.7° (c=1.0, water). The optical purity of this product was 98.0% by high-performance liquid chromatographic analysis.

Reference Example 2

A hot soloution of 11.19 g of (±)-erythro-2-amino-1-(2-ethoxyphenyl)-1-propanol (content of the erythro-form, 99% or more) in 15 ml of methanol was added to a hot solution of 8.60 g of L-(+)-tartaric acid in 20 ml of methanol, and the resulting solution was allowed to cool. The deposited crystals were collected by filtration to obtain 10.44 g of the L-(+)-tartaric acid salt of (−)-erythro-2-amino-1-(2-ethoxyphenyl)-1-propanol. $[\alpha]_D$: +6.3° (c=1.0, water). This salt was recrystallized from methanol to obtain 4.50 g of crystals. $[\alpha]_D$: −8.4° (c=1.0, water). The crystals were decomposed with a 20% aqueous sodium hydroxide solution and extracted with chloroform to obtain 2.79 g of (−)-erythro-2-amino-1-(2-ethoxyphenyl)-1-propanol. $[\alpha]_D$: −16.4° (c=1.0, $CHCl_3$). This aminoalcohol was converted to its sugar derivative (diastereomer) and analyzed by high-performance liquid chromatography to find that its optical purity was 63.2%. By dissolving this aminoalcohol in diethyl ether-chloroform mixture and passing hydrogen chloride gas therethrough, 3.18 g of (−)-erythro-2-amino-1-(2-ethoxyphenyl)-1-propanol hydrochloride was obtained. Recrystallization of this product from isopropanol was repeated four times to obtain 1.02 g of crystals. $[\alpha]_D$: −43.6° (c=1.0, water). The optical purity of the crystals was 97.8%.

Reference Example 3

On adding a solution of 4.56 g (0.0263 mole) of N-acetyl-L-leucine and 1.11 g (0.0263 mole) of 95% sodium hydroxide in 50 ml of water to a solution of 12.17 g (0.0525 mole) of (±)-erythro-2-amino-(2-methoxy-5-methylphenyl)-propanol hydrochloride (erythro-form, 99% or more) in 115 ml of water, crystals were deposited. The crystals were re-dissolved in the solution by additionally adding 500 ml of water, and after allowing to cool, the deposited crystals were collected by filtration. The yield of the N-acetyl-L-leucine salt of (−)-erythro-2-amino-1-(2-methoxy-5-methylphenyl)-1-propanol was 3.63 g. $[\alpha]_D$: −29.2° (c=1.0, water). This salt was decomposed with a 10% aqueous sodium hydroxide solution and extracted with chloroform to obtain 1.94 g of (−)-erythro-2-amino-1-(2-methoxy-5-methylphenyl)-1-propanol. $[\alpha]_D$: −22.1° (c=1.1, $CHCl_3$). By dissolving this product in diethyl ether and passing hydrogen chloride gas therethrough, 2.13 g of (−)-erythro-2-amino-1-(2-methoxy-5-methylphenyl)-1-propanol hydrochloride was obtained. This salt was recrystallized from isopropanol to obtain 1.65 g of crystals. $[\alpha]_D$: −22.2° (c=1.0, water). The optical purity of this product as sugar derivative (diasteromer) was 97.8% by high-performance liquid chromatographic analysis.

Reference Example 4

20 Grams (0.0938 mole) of 2-amino-1-(2-ethoxyphenyl)-1-propanone hydrochloride was dissolved in 300 ml of water, and 1.77 g (0.0468 mole) of sodium borohydride was added at 5° to 8°C. After maintaining the temperature for 1 hour with stirring, the reaction solution was allowed to stand overnight at room temperature. Thereafter, the reaction solution was acidified with conc. hydrochloric acid and then made alkaline with a 25% aqueous sodium hydroxide solution. The deposited crystals were collected by filtration, washed with water and dried to obtain 13.40 g of erythro-2-amino-1-(2-ethoxyphenyl)-1-propanol as crystals (erythro/threo=98.4/1.6). This was recrystallized from toluene to obtain 12.39 g of crystals (content of the erythro-form, 99% or more). m.p., 89—91°C.

NMR spectrum ($CDCl_3$): δ (ppm): 0.99 (d, 3H), 1.40 (t, 3H), 1.6—2.4 (broad 2H), 3.24 (m, H), 3.99 (q, 2H), 4.76 (d, H), 6.7—7.04 (m, 2H), 7.05—7.45 (m, 2H).

**Claims**

1. An asymmetrically modified boron hydride type compound obtainable by reacting an optically active amino alcohol of the formula:

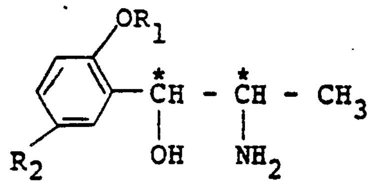

(I)

wherein $R_1$ is a $C_{1-3}$ alkyl group, $R_2$ is a hydrogen atom, a $C_{1-3}$ alkoxyl group or a $C_{1-4}$ alkyl group, and a mark * means an asymmetric carbon with the proviso that $R_2$ is not an ethoxy group when $R_1$ is an ethyl group, or its salt with an acid selected from the group consisting of mineral acids, carboxylic acids and organic sulfonic acids, with a boron hydride compound.

2. A compound according to Claim 1, wherein, in the above formula (I), $R_1$ is a methyl group or an ethyl group and $R_2$ is a hydrogen atom, a methoxy group or a methyl group.

3. A compound according to Claim 1 or 2, wherein the boron hydride compound is a metal borohydride.

4. A compound according to Claim 3, wherein the metal boronhydride is sodium borohydride, potassium borohydride, lithium borohydride or zinc borohydride.

5. A compound according to Claim 3 or 4, wherein the molar ratio of the salt of the optically active amino alcohol to the metal borohydride is 1:0.7 to 1:1.3, as converted to boron basis.

6. A compound according to Claim 1 or 2, wherein the boron hydride compound is a borane.

7. A compound according to Claim 6, wherein the molar ratio of the optically active amino alcohol to the borane is 1:0.7 to 1:1.3, as converted to boron basis.

8. A method for producing an asymmetrically modified boron hydride type compound, characterized in that an optically active amino alcohol of the formula:

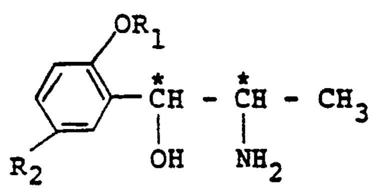

(I)

wherein $R^1$ is a $C_{1-3}$ alkyl group, $R_2$ is a hydrogen atom, a $C_{1-3}$ alkoxyl group or a $C_{1-4}$ alkyl group and a mark * means an asymmetric carbon with the proviso that $R_2$ is not an ethoxy group when $R_1$ is an ethyl group, or its salt with an acid selected from the group consisting of mineral acids, carboxylic acids and organic sulfonic acids, with a boron hydride compound.

9. A method according to Claim 8, wherein, in the above formula (I), $R_1$ is a methyl group or an ethyl group and $R_2$ is a hydrogen atom, a methoxy group or a methyl group.

10. A method according to Claim 8 or 9, wherein the boron hydride compound is a metal borohydride.

11. A method according to Claim 10, wherein the metal borohydride is sodium borohydride, potassium borohydride, lithium borohydride or zinc borohydride.

12. A method according to Claim 10 or 11, wherein the molar ratio of the salt of the optically active amino alcohol to the metal borohydride is 1:0.7 to 1:1.3, as converted to boron basis.

13. A method according to Claim 8 or 9, wherein the boron hydride compound is a borane.

14. A method according to Claim 13, wherein the molar ratio of the optically active amino alcohol to the borane is 1:0.7 to 1:1.3, as converted to boron basis.

15. A method for producing an optically active alcohol derivative of the formula:

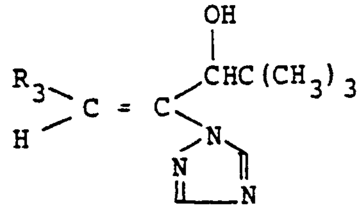

(II)

wherein $R_3$ is a cycloalkyl group or a phenyl group which may be substituted with at least one of halogen, a

mark * means an asymmetric carbon, characterized in that the asymmetric reduction of a ketone compound of the formula:

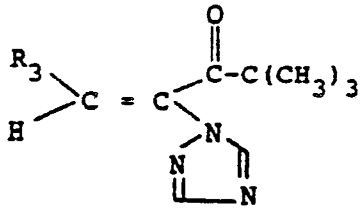

(III)

wherein $R_3$ has the same meaning as above, is carried out with an asymmetrically modified boron hydride type compound obtainable by reacting an optically active amino alcohol of the formula:

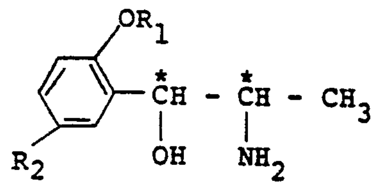

(I)

wherein $R_1$ is a $C_{1-3}$ alkyl group, $R_2$ is a hydrogen atom, a $C_{1-3}$ alkoxyl group or a $C_{1-4}$ alkyl group, and a mark * has the same meaning as above with the proviso that $R_2$ is not an ethoxy group when $R_1$ is an ethyl group, or its salt with an acid selected from the group consisting of mineral acids, carboxylic acids and organic sulfonic acids, with a boron hydride compound in the presence or absence of an acid.

16. A method according to Claim 15, wherein, in the above formulae (II) and (III), $R_3$ is a 2,4-dichlorophenyl, 4-chlorophenyl or cyclohexyl group.

17. A method according to Claim 15 or 16, wherein, in the above formula (I), $R_1$ is a methyl group or an ethyl group and $R_2$ is a hydrogen atom, a methoxy group or a methyl group.

18. A method according to any of Claims 15 to 17, wherein the boron hydride compound is a metal borohydride.

19. A method according to Claim 18, wherein the metal borohydride is sodium borohydride, potassium borohydride, lithium borohydride or zinc borohydride.

20. A method according to Claim 18 or 19, wherein the molar ratio of the salt of the optically active amino alcohol to the metal borohydride is 1:0.7 to 1:1.3, as converted to boron basis.

21. A method according to any of Claims 15 to 17, wherein the boron hydride compound is a borane.

22. A method according to Claim 21, wherein the molar ratio of the optically active amino alcohol to the borane is 1:0.7 to 1:1.3, as converted to boron basis.

23. A method according to Claim 18, 19 or 20, wherein the asymmetric reduction is carried out in the presence of an acid.

24. A method according to Claim 23, wherein the acid is a Lewis acid, organic acid or mineral acid.

**Patentansprüche**

1. Asymmetrisch modifizierte Verbindung vom Borhydrid-Typ, erhältlich durch Umsetzung eines optisch aktiven Aminoalkohols der Formel

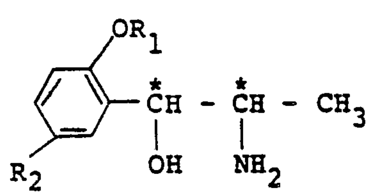

(I)

wobei $R_1$ einen $C_{1-3}$-Alkylrest bedeutet, $R_2$ ein Wasserstoffatom, einen $C_{1-3}$-Alkoxyrest oder einen $C_{1-4}$-Alkylrest darstellt, und das Zeichen * ein asymmetrisches Kohlenstoffatom bedeutet, mit der Maßgabe, daß $R_2$ keine Äthoxygruppe ist, wenn $R_1$ eine Äthylgruppe bedeutet, oder ihr Salz mit einer Säure, ausgewählt aus der Gruppe der Mineralsäuren, Carbonsäuren und organischen Sulfonsäuren, mit einer Borhydrid-Verbindung.

2. Verbindung nach Anspruch 1, wobei in der vorstehenden Formel (I) $R_1$ eine Methylgruppe oder eine Äthylgruppe bedeutet und $R_2$ ein Wasserstoffatom, eine Methoxygruppe oder eine Methylgruppe darstellt.

3. Verbindung nach Anspruch 1 oder 2, wobei die Borhydrid-Verbindung ein Metallborhydrid ist.

4. Verbindung nach Anspruch 3, wobei das Metallborhydrid Natriumborhydrid, Kaliumborhydrid, Lithiumborhydrid oder Zinkborhydrid ist.

5. Verbindung nach Anspruch 3 oder 4, wobei das Molverhältnis des Salzes des optisch aktiven Aminoalkohols zu dem Metallborhydrid 1:0,7 bis 1:1,3, umgewandelt auf Borbasis, ist.

6. Verbindung nach Anspruch 1 oder 2, wobei die Borhydrid-Verbindung ein Boran ist.

7. Verbindung nach Anspruch 6, wobei das Molverhältnis des optisch aktiven Aminoalkohols zu dem Boran 1:0,7 bis 1:1,3, umgewandelt auf Borbasis, ist.

8. Verfahren zur Herstellung einer asymmetrisch modifizierten Verbindung vom Borhydrid-Typ, dadurch gekennzeichnet, daß ein optisch aktiver Aminoalkohol der Formel

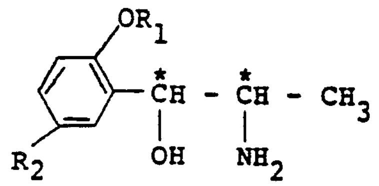

(I)

wobei $R_1$ einen $C_{1-3}$-Alkylrest bedeutet, $R_2$ ein Wasserstoffatom, einen $C_{1-3}$-Alkoxyrest oder einen $C_{1-4}$-Alkylrest darstellt, und das Zeichen * ein asymmetrisches Kohlenstoffatom bedeutet, mit der Maßgabe, daß $R_2$ keine Äthoxygruppe ist, wenn $R_1$ eine Äthylgruppe bedeutet, oder ihr Salz mit einer Säure, ausgewählt aus der Gruppe der Mineralsäuren, Carbonsäuren und organischen Sulfonsäuren, mit einer Borhydrid-Verbindung umgesetzt wird.

9. Verfahren nach Anspruch 8, wobei in der vorstehenden Formel (I) $R_1$ eine Methylgruppe oder eine Äthylgruppe bedeutet und $R_2$ ein Wasserstoffatom, eine Methoxygruppe oder eine Methylgruppe darstellt.

10. Verfahren nach Anspruch 8 oder 9, wobei die Borhydrid-Verbindung ein Metallborhydrid ist.

11. Verfahren nach Anspruch 10, wobei das Metallborhydrid Natriumborhydrid, Kaliumborhydrid, Lithiumborhydrid oder Zinkborhydrid ist.

12. Verfahren nach Anspruch 10 oder 11, wobei das Molverhältnis des Salzes des optisch aktiven Aminoalkohols zu dem Metallborhydrid 1:0,7 bis 1:1,3, umgewandelt auf Borbasis, ist.

13. Verfahren nach Anspruch 8 oder 9, wobei die Borhydrid-Verbindung ein Boran ist.

14. Verfahren nach Anspruch 13, wobei das Molverhältnis des optisch aktiven Aminoalkohols zu dem Boran 1:0,7 bis 1:1,3, umgewandelt auf Borbasis, ist.

15. Verfahren zur Herstellung eines optisch aktiven Alkohol-Derivates der Formel

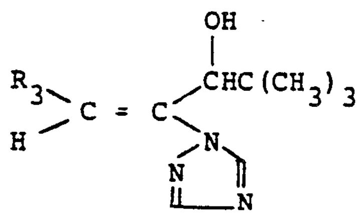

(II)

wobei $R_3$ einen Cycloalkylrest oder eine Phenylgruppe bedeutet, welche durch mindestens ein Halogenatom substitiert sein kann und das Zeichen * ein asymmetrisches Kohlenstoffatom bedeutet, dadurch gekennzeichnet, daß die asymmetrische Reduktion einer Keton-Verbindung der Formel

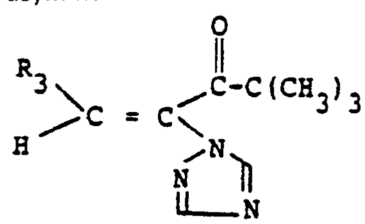

(III)

wobei $R_3$ die vorstehende Bedeutung hat, mit einer asymmetrisch modifizierten Verbindung vom Borhydrid-Typ ausgeführt wird, die erhältlich ist durch Umsetzung eines optisch aktiven Aminoalkohols der Formel

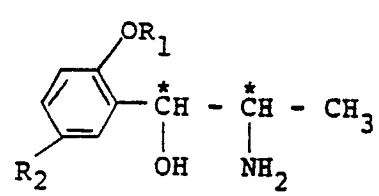

(I)

wobei $R_1$ einen $C_{1-3}$-Alkylrest bedeutet, $R_2$ ein Wasserstoffatom, einen $C_{1-3}$-Alkoxyrest oder einen $C_{1-4}$-Alkylrest darstellt, und das Zeichen * ein asymmetrisches Kohlenstoffatom bedeutet, mit der Maßgabe, daß $R_2$ keine Äthoxygruppe ist, wenn $R_1$ eine Äthylgruppe bedeutet, oder ihr Salz mit einer Säure,

ausgewählt aus der Gruppe der Mineralsäuren, Carbonsäuren und organischen Sulfonsäuren, mit einer Borhydrid-Verbindung in Gegenwart oder Abwesenheit einer Säure.

16. Verfahren nach Anspruch 15, wobei in den vorstehenden Formeln (II) und (III) $R_3$ eine 2,4-Dichlorphenyl-, 4-Chlorphenyl- oder Cyclohexylgruppe bedeutet.

17. Verfahren nach Anspruch 15 oder 16, wobei in der vorstehenden Formel (I) $R_1$ eine Methylgruppe oder eine Äthylgruppe bedeutet und $R_2$ ein Wasserstoffatom, eine Methoxygruppe oder eine Methylgruppe darstellt.

18. Verfahren nach einem der Ansprüche 15 bis 17, wobei die Borhydrid-Verbindung ein Metallborhydrid ist.

19. Verfahren nach Anspruch 18, wobei das Metallborhydrid Natriumborhydrid, Kaliumborhydrid, Lithiumborhydrid oder Zinkborhydrid ist.

20. Verfahren nach Anspruch 18 oder 19, wobei das Molverhältnis des Salzes des optisch aktiven Aminoalkohols zu dem Metallborhydrid 1:0,7 bis 1:1,3, umgewandelt auf Borbasis, ist.

21. Verfahren nach einem der Ansprüche 15 bis 17, wobie die Borhydrid-Verbindung ein Boran ist.

22. Verfahren nach Anspruch 21, wobei das Molverhältnis des optisch aktiven Aminoalkohols zu dem Boran 1:0,7 bis 1:1,3, umgewandelt auf Borbasis, ist.

23. Verfahren nach Anspruch 18, 19 oder 20, wobei die asymmetrische Reduktion in Gegenwart einer Säure durchgeführt wird.

24. Verfahren nach Anspruch 23, wobei die Säure eine Lewis-Säure, organische Säure oder Mineralsäure ist.

**Revendications**

1. Composé de type hydrure de bore modifié de façon asymétrique qui peut être obtenu par réaction d'un amino-alcool optiquement actif de formule

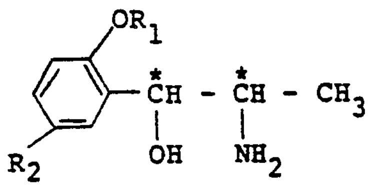

(I)

dans laquelle $R_1$ est un groupement alkyle en $C_1$—$C_3$, $R_2$ est un atome d'hydrogène ou un groupement alcoxy en $C_1$—$C_3$ ou alkyle en $C_1$—$C_4$, et une marque * indique un carbone asymétrique pourvu que $R_2$ ne soit pas un groupement méthoxy quand $R_1$ est un groupement éthyle, ou son sel formé avec un acide choisi dans le groupe contenant les acides minéraux, les acides carboxyliques et les acides sulfoniques organiques, avec un composé hydrure de bore.

2. Composé selon la revendication 1, dans lequel, dans la formule précédente (I), $R_1$ est un groupement méthyle ou n groupement éthyle et $R_2$ est un atome d'hydrogène, un groupement méthoxy ou un groupement méthyle.

3. Composé selon la revendication 1 ou 2, dans lequel le composé hydrure de bore est un borohydrure métallique.

4. Composé selon la revendication 3, dans lequel le borohydrure métallique est le borohydrure de sodium, le borohydrure de potassium, le borohydrure de lithium ou le borohydure de zinc.

5. Composé selon la revendication 3 ou 4, dans lequel le rapport molaire du sel d'amino-alcool optiquement actif au borohydrure métallique est 1:0,7 à 1:1,3, ramené au bore.

6. Composé selon la revendication 1 ou 2, dans lequel le composé hydrure de bore est un borane.

7. Composé selon la revendication 6, dans lequel le rapport molaire de l'amino-alcool optiquement actif au borane est 1:0,7 à 1:1,3, ramené au bore.

8. Procédé de préparation d'un composé du type hydrure de bore modifié de façon asymétrique, caractérisé en ce qu'on fait réagir avec un composé hydrure de bore un amino-alcool optiquement actif de formule

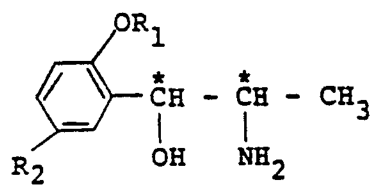

(I)

dans laquelle $R_1$ est un groupement alkyle en $C_1$—$C_3$, $R_2$ est un atome d'hydrogène ou un groupement alcoxy en $C_1$—$C_3$ ou alkyle en $C_1$—$C_4$, et une marque * indique in carbone asymétrique pourvu que $R_2$ ne soit pas un groupement éthoxy quand $R_1$ est un groupement éthyle, ou son sel formé avec un acide choisi dans le groupe comprenant les acides minéraux, les acides carboxyliques et les acides sulfoniques organiques.

9. Procédé selon la revendication 8, dans lequel, dans la formule précédente (I), $R_1$ est un groupement méthyle ou un groupement éthyle et $R_2$ est un atome d'hydrogène ou un groupement méthoxy ou méthyle.

10. Procédé selon la revendication 8 ou 9, dans lequel le composé hydrure de bore est un borohydrure métallique.

11. Procédé selon la revendication 10, dans lequel le borohydrure métallique est le borohydrure de sodium, le borohydrure de potassium, le borohydrure de lithium ou le borohydrure de zinc.

12. Procédé selon la revendication 10 ou 11, dans lequel le rapport molaire du sel d'amino-alcool optiquement actif au borohydrure métallique est 1:0,7 à 1:1,3, ramené au bore.

13. Procédé selon la revendication 8 ou 9, dans lequel le composé hydrure de bore est un borane.

14. Procédé selon la revendication 13, dans lequel le rapport molaire de l'amino-alcool optiquement actif au borane est 1:0,7 à 1:1,3, ramené au bore.

15. Procédé de préparation d'une dérivé alcoolique optiquement actif de formule

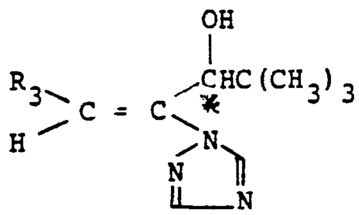

(II)

dans laquelle $R_3$ est un groupement cycloalkyle ou un groupement phényle qui peut être substitué par au moins un des halogènes, une marque * indique un carbone asymétrique, caractérisé en ce qu'on effectue, en présence ou en l'absence d'un acide, la réduction asymétrique d'un composé cétonique de formule

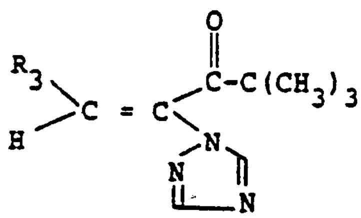

(III)

dans laquelle $R_3$ est tel que défini précédemment, avec un composé de type hydrure de bore modifié asymétriquement qui peut être obtenu par réaction d'un amino-alcool optiquement actif de formule

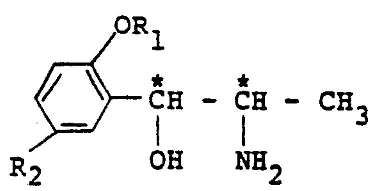

(I)

dans laquelle $R_1$ est un groupement alkyle en $C_1$—$C_3$, $R_2$ est un atome d'hydrogène ou un groupement alcoxy en $C_1$—$C_3$ ou alkyle en $C_1$—$C_4$, et une marque * indique in carbone asymétrique pourvu que $R_2$ ne soit pas un groupement éthoxy quand $R_1$ est un groupement éthyle, ou son sel avec un acide choisi dans le groupe comprenant les acides minéraux, les acides carboxyliques et les acides sulfoniques organiques, avec un composé hydrure de bore.

16. Procédé selon la revendication 15, dans lequel, dans les formules (II) et (III) précédentes, $R_3$ est un groupement 2,4-dichlorophényle, 4-chlorophényle ou cyclohexyle.

17. Procédé selon la revendication 15 ou 16, dans lequel, dans la formule précédente (I), $R_1$ est un groupement méthyle ou éthyle et $R_2$ est un atome d'hydrogène ou un groupement méthoxy ou méthyl.

18. Procédé selon l'une quelconque des revendications 15 à 17, dans lequel le composé hydrure de bore est un borohydrure métallique.

19. Procédé selon la revendication 18, dans lequel le borohydrure métallique est le borohydrure de sodium, le borohydrure de potassium, le borohydrure de lithium ou le borohydrure de zinc.

20. Procédé selon la revendication 18 ou 19, dans lequel le rapport molaire du sel d'amino-alcool optiquement actif au borohydrure métallique est 1:0,7 à 1:1,3, ramené au bore.

21. Procédé selon l'une quelconque des revendications 15 à 17, dans lequel le composé hydrure de bore est un borane.

22. Procédé selon la revendication 21, dans lequel le rapport molaire de l'amino-alcool optiquement actif au borane est 1:0,7 à 1:1,3, ramené au bore.

23. Procédé selon l'une des revendications 18, 19 ou 20, dans lequel la réduction asymétrique est effectuée en présence d'un acide.

24. Procédé selon la revendication 23, dans lequel l'acide est un acide de Lewis, un acide organique ou un acide minéral.